(19) **Europäisches Patentamt / European Patent Office / Office européen des brevets**

(11) **EP 1 958 953 A1**

(12) **EUROPEAN PATENT APPLICATION**
published in accordance with Art. 158(3) EPC

(43) Date of publication:
**20.08.2008 Bulletin 2008/34**

(21) Application number: **06833735.1**

(22) Date of filing: **30.11.2006**

(51) Int Cl.:
*C07H 5/02* (2006.01)    *A61K 51/00* (2006.01)
*C07B 61/00* (2006.01)

(86) International application number:
**PCT/JP2006/323932**

(87) International publication number:
**WO 2007/063940 (07.06.2007 Gazette 2007/23)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **02.12.2005 JP 2005349091**

(71) Applicant: **NIHON MEDI-PHYSICS CO., LTD.
Tokyo 136-0075 (JP)**

(72) Inventors:
• **HIRANO, Keiichi
Sodegaura-shi Chiba 2990266 (JP)**
• **NAKAMURA, Daisaku
Sodegaura-shi Chiba 2990266 (JP)**

(74) Representative: **Wilhelms · Kilian & Partner
Patentanwälte
Eduard-Schmid-Strasse 2
81541 München (DE)**

(54) **PROCESS FOR PRODUCTION OF COMPOUND LABELED WITH RADIOACTIVE FLUORINE**

(57) A process for production of [18F]-FDG is provided, which can produce [18F]-FDG in a high and stable yield of synthesis. A process for production of a compound labeled with radioactive fluorine comprises the steps of: preparing a reaction solution by adding, under a hermetic condition, TATM and an inert organic solvent to a mixture containing [[18F]]fluoride ions, a phase transfer catalyst and potassium ions; giving a reaction condition to the reaction solution under a hermetic condition to obtain [18F]-TAFDG; and subjecting the obtained [18F]-TAFDG to a deprotection process and optionally to a purification process to obtain [18F]-FDG.

**EP 1 958 953 A1**

**EP 1 958 953 A1**

**Description**

TECHNICAL FIELD

The present invention relates a process for production of a compound labeled with radioactive fluorine, specifically 2-[18F]fluoro-2-deoxy-D-glucose.

BACKGROUND ART

Nuclear medicine examination represented by positron emission tomography (hereinafter referred to as PET) and single photon emission computed tomography (hereinafter referred to as SPECT) is effective in diagnosing a variety of diseases including cancers. These examination techniques involve administrating an agent labeled with a specific radioisotope (hereinafter referred to as radiopharmaceutical) to a patient, followed by detecting γ-ray emitted directly or indirectly from the agent. Nuclear medicine examination is characteristic in that it has not only high specificity and sensitivity to diseases but also an advantage of providing information on the function of lesions, compared to other examination techniques.

For example, 2-[18F]fluoro-2-deoxy-D-glucose (hereinafter referred to as 18F-FDG), one of the radiopharmaceuticals used for PET examination, tends to be concentrated in areas where glucose metabolism is enhanced, thereby making it possible to specifically detect tumors in which glucose metabolism is enhanced.

Among the above-mentioned nuclear medicine examinations, PET provides images of higher quality and thus can provide images higher in diagnosis performance, compared to SPECT that has been widely used conventionally in clinical use. PET examination is, therefore, expected as a new diagnostic modality succeeding SPECT examination, and development of radiopharmaceuticals for PET examination (hereinafter referred to as PET diagnostic agent) is now carried out in many research facilities and the like. For example, various receptor mapping agents and blood flow diagnostic agents have been synthesized and are under investigation for clinical application.

The PET diagnostic agent is an agent that contains, as an effective component, a compound labeled with a positron emitting nuclide such as 11C, 15O and 18F. Among PET diagnostic agents, organic compounds labeled with 18F represented by 18F-FDG are most widely used as they possess a nuclide longer in half-life than other nuclides for PET. There have been reported various methods for production of the representative compound, 18F-FDG, and most of them are generally classified into a method proposed by Hamacher (hereinafter referred to as Hamacher method) and an on-column method.

The Hamacher method is a method wherein [18F]fluoride ions are activated by evaporating a solution containing [18F] fluoride ions, potassium carbonate and a phase transfer catalyst to dryness, then a solution in acetonitrile of 1,3,4,6-tetra-O-acetyl-2-O-trifluoromethanesulfonyl-β-D-mannopyranose (hereinafter referred to as TATM) that is a labeling precursor is added and heated to give an intermediate, 1,3,4,6-tetra-O-acetyl-2-fluoro-2-deoxyglucose (hereinafter referred to as 18F-TAFDG), followed by subjecting the intermediate to a deprotection process and a purification process to give targeted 18F-FDG. On the other hand, the on-column method is a method wherein 18F-FDG is obtained by performing activation of [18F]fluoride ions and 18F labeling reaction in a column followed by deprotection and purification of the resultant labeled product (see Patent Document 1, Non-Patent Document 1 and Non-Patent Document 2).

Thus, when the synthesis of 18F-FDG is performed by the Hamacher method, the reaction solution to be provided for [18F]-fluorination reaction is prepared by adding a solution of TATM as a labeling precursor in acetonitrile to a mixture containing activated [18F]fluoride ions. This procedure has been usually performed under normal pressure, for example, in a given vessel with an exit valve thereof being open (see, for example, Non-Patent Document 3).

Patent Document 1: Japanese Patent Laid-Open (Kokai) No. 6-157572.

Non-Patent Document 1: Hamacher K., Coenen H.H., Stocklin G., "Efficient Stereospecific Synthesis of No-carrier-added-2-[18F]fluoro-2-deoxy-D-glucose Using Aminopolyether Supported Nucleophilic Substitution, "J. Nucl. Med., 1986, 27, 2, p.235-238.

Non-Patent Document 2: K. Hamacher et al., "Computer-aided Synthesis (CAS) of No-carrier-added-2-[18F]Fluoro-2-deoxy-D-glucose: an Efficient Automated System for the Aminopolyether-supported Nucleophilic Fluorination" Applied Radiation and Isotopes, (Great Britain), Pergamon Press, 1990, 41, 1, p.49-55.

Non-Patent Document 3: David L. Alexoff et al., "Ion Chromatographic Analysis of High Specific Activity 18FDG Preparations and Detection of the Chemical Impurity 2-Deoxy-2-chloro-D-glucose.", Applied Radiation and Isotopes, (Great Britain), Pergamon Press, 1992, 43, 11, p.1313-1322.

DISCLOSURE OF THE INVENTION

PROBLEM TO BE SOLVED BY THE INVENTION

Among the representative methods for producing $^{18}$F-FDG, the Hamacher method has a feature such that it is simple and convenient and can be easily applied to an automated synthesis apparatus, but nevertheless has a problem such that production yield may greatly vary in some cases. In order to supply $^{18}$F-FDG commercially, it is desirable to adopt a method or condition that enables the compound to be produced in higher yield more stably.

The present invention has been made in light of the above situation, and has aimed at providing a process for production of $^{18}$F-FDG, which can stably achieve a high yield of radiofluorination.

MEANS FOR SOLVING THE PROBLEM

As a result of diligent studies, the inventors have completed the invention by finding that the above problem can solved and $^{18}$F-FDG can be produced with higher yield more stably by performing, under a hermetic condition, the step of adding TATM as the labeling precursor to the mixture containing activated [$^{18}$F]fluoride ions. Conventionally, it has been common that the raw materials are added to the reaction vessel in the so-called open system from the viewpoint of preventing an excessive increase of internal pressure in the reaction vessel. Unlike such conventional methods, the inventors have found that the yield of $^{18}$F-FDG is improved by adding raw materials and the like to the reaction vessel in a hermetic state, thereby completing the invention.

The process for production of a compound labeled with radioactive fluorine according to the present invention comprises the steps of: preparing a reaction solution by adding, under a hermetic condition, TATM and an inert organic solvent to a mixture containing [$^{18}$F]fluoride ions, a phase transfer catalyst and potassium ions; giving a reaction condition to said reaction solution under a hermetic condition to obtain $^{18}$F-TAFDG; and subjecting the obtained $^{18}$F-TAFDG to a deprotection process and optionally a purification process to obtain $^{18}$F-FDG.

"Hermetic condition" used herein refers to a condition under which incomings and outgoings of solid, liquid and gas are substantially blocked in a reaction vessel except inflow of raw materials and the like resulting from operations for adding raw materials such as TATM and an inert organic solvent. Thus, the addition of TATM and an inert organic solvent under a hermetic condition means providing a reaction vessel in which all flow paths are closed except the flow path for raw materials such as TATM and the like or a reaction vessel which has no aperture other than the aperture for introduction of raw materials such as TATM and the like, and adding raw materials such as TATM, an inert organic and the like to the reaction vessel through the above-mentioned flow path or aperture for the raw materials.

For example, in the case where a reaction vessel with a plurality of flow paths is used, raw materials such as TATM are introduced into the reaction vessel in a state in which all flow paths are closed except the flow paths for introduction of the raw materials such as TATM so that the flow of gas and the like from the vessel to outside is substantially blocked. On the other hand, in the case where a reaction vessel connected with a single path or a reaction vessel with only one aperture connected with an inlet pipe is used, raw materials such as TATM are introduced into the reaction vessel through this flow path or inlet pipe.

And, $^{18}$F-TAFDG is obtained by providing a condition sufficient to allow the [$^{18}$F]-fluorination reaction of TATM to proceed by means of heating or the like under a hermetic condition which is created by closing all the flow paths or the single flow path or aperture (hereinafter collectively referred to as "flow inlet") of the reaction vessel.

That is to say, in accordance with an aspect of the production process of the present invention, there is provided a process comprising the steps of: obtaining a mixture containing [$^{18}$F]fluoride ions, a phase transfer catalyst and potassium ions in a vessel connected with one or more flow paths; adding TATM and an inert organic solvent to the mixture through a flow path for introducing raw materials in a state in which the flow paths other than the flow path for introducing raw materials are closed to prepare a reaction solution; heating the reaction solution under a hermetic condition in a state in which all the flow paths of the vessel are closed to obtain $^{18}$F-TAFDG; and subjecting the obtained $^{18}$F-TAFDG to a deprotection process to obtain $^{18}$F-FDG.

According to still another aspect of the production process of the present invention, there is provided a process comprising the steps of: obtaining a mixture containing [$^{18}$F]fluoride ions, a phase transfer catalyst and potassium ions in a vessel having one aperture; adding TATM and an inert organic solvent to the mixture through the aperture to prepare a reaction solution; heating the reaction solution under a hermetic condition in a state in which the aperture is closed to obtain $^{18}$F-TAFDG; and subjecting the obtained $^{18}$F-TAFDG to a deprotection process to obtain $^{18}$F-FDG.

The mixture containing [$^{18}$F] fluoride ions, a phase transfer catalyst and potassium ions can be obtained by various methods. For example, a method may be used wherein $H_2$$^{18}$O enriched water containing [$^{18}$F]fluoride ions is passed through an anion-exchange column to collect radioactive fluoride by adsorption to the column, then a potassium carbonate solution is allowed to pass through the column to elute [$^{18}$F]fluoride ions, and the eluate is supplemented with a phase transfer catalyst and is evaporated to dryness.

In the present invention, various methods may be used as a method for adding TATM in the step of preparing a reaction solution. For example, a method may be used wherein TATM is added in a form of a solution in which TATM is dissolved in an inert organic solvent. Various methods may be used as a method for running such a TATM solution into a reaction vessel. For example, it is possible to use a method wherein a TATM solution is prepared in a reagent vessel and introduced into a reaction vessel through a flow inlet by pressurizing the reagent vessel with an inert gas, or a method wherein a reaction vessel is kept under negative pressure beforehand by means of degassing and the like, and then the TATM solution is drawn into the vessel through the flow inlet.

As an inert organic solvent, various solvents may be used which have no reactivity with [18F]fluoride ions, a phase transfer catalyst, potassium ions and TATM; acetonitrile may preferably be used. The organic solvent may be an anhydrate. Alternatively, the organic solvent may contain a certain amount of water if it is an amphipathic solvent such as acetonitrile.

As the deprotection process for 18F-TAFDG, known methods can be used, for example, a method described in the literature "Manufacture and Quality Control of Radioactive Agents for PET (PET-you Houshasei Yakuzai no Seizou oyobi Hinshitu Kanri in Japanese) - A Guideline to Synthesis and Clinical Use - 2nd Ed." edited by PET Kagaku Workshop. Specific examples of the method include, for example, an acid hydrolysis method in which hydrochloric acid is added to 18F-TAFDG followed by heating, and an alkaline hydrolysis method in which sodium hydroxide is added.

Meanwhile, the process according to the present invention may be one in which a purification process is further performed following the deprotection process. As the purification process in this case, known methods can be used ("Manufacture and Quality Control of Radioactive Agents for PET (PET-you Houshasei Yakuzai no Seizou oyobi Hinshitu Kanri in Japanese) - A Guideline to Synthesis and Clinical Use - 2nd Ed." edited by PET Kagaku Workshop). As an example, a method may be exemplified wherein a series of purification columns composed of a reverse phase column, an alumina column and the like are used.

EFFECT OF THE INVENTION

The use of the process according to the present invention enables stable production of 18F-FDG in high yield.

BEST MODE FOR CARRYING OUT THE INVENTION

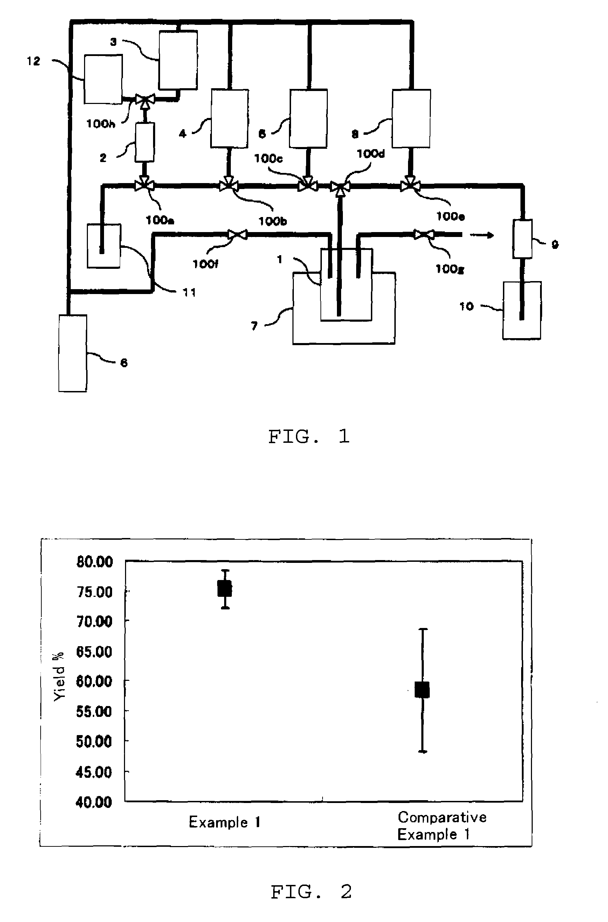

Hereinafter, the most preferable embodiment will be described about the process for production of a compound labeled with radioactive fluorine according to the present invention with reference to drawings. Figure 1 shows an example of an apparatus for performing the production process according to the present invention. The apparatus of Figure 1 is composed of a reaction vessel (1), reagent vessels for stocking necessary reagents and raw materials ((vessel (3), vessel (4), vessel (5) and vessel (8)), an anion exchange column (2), a purification column (9), an 18F-FDG collecting vessel (10), an 18O enriched water recovering vessel (11), a helium cylinder (6), a heating apparatus (7) and an 18F storing vessel (12). In Figure 1, the vessel (3), the vessel (4), the vessel (5) and the vessel (8) are charged with a potassium carbonate solution, a phase transfer catalyst, a solution of TATM in acetonitrile and hydrochloric acid, respectively.

In a process for production of 18F-FDG according to the present invention, first, a mixture containing a phase transfer catalyst, [18F]fluoride ions and potassium ions is obtained in the reaction vessel (1). [18F]fluoride ions can be obtained by a known method, for example, a method in which $H_2^{18}O$ enriched water is used as a target and exposed to a proton bombardment. In this instance, [18F]fluoride ions exist in the $H_2^{18}O$ enriched water used as a target. This $H_2^{18}O$ enriched water containing [18F]fluoride ions is passed from the vessel (12) through a three-way stopcock (100h) to the anion exchange column (2), and then recovered through a three-way stopcock (100a) into the vessel (11). By this operation, [18F]fluoride ions can be collected by adsorption to the column and can be separated from the $H_2^{18}O$ enriched water which is recovered in the vessel (11). Then, a valve (100g) is opened and the reaction vessel (1) is connected to the outlet side of the column (2) by operating three-way stopcocks (100a, 100b, 100c and 100d). In this state, the potassium carbonate solution is run from the vessel (3) to the column (2) to elute [18F]fluoride ions into the reaction vessel (1), then the flow path between the vessel (1) and each vessel is closed by operating the three-way stopcock (100a). Then, the vessel (4) and the reaction vessel (1) are connected with each other by operating the three-way stopcock (100b), and the phase transfer catalyst from the vessel (4) is added to the reaction vessel (1). The flow path between the vessel (1) and the vessel (4) is closed by operating the three-way stopcock (100d), followed by heating the reaction vessel (1) using the heating apparatus (7) with the valve (100g) being open to exsiccate. By these series of operation, a mixture containing a phase transfer catalyst, [18F]fluoride ions and potassium ions can be obtained in the vessel (1).

Amount of potassium carbonate to be used here may be about 0.3 or more in terms of molar ratio relative to the labeling precursor TATM, but should not be excessive because it causes decrease of yield unfavorably. In a preferable aspect, concentration and amount of the potassium carbonate solution are adjusted so that the molar ratio of potassium ion to TATM is 0.3 to 4.

Various compounds having a property to form a clathrate with $^{18}F$ ion may be used as a phase transfer catalyst. Specifically, various compounds commonly used for production of organic compounds labeled with radioactive fluorine may be used; 18-crown-6-ether and other various aminopolyethers may be used. In the most preferable aspect, CRYPTOFIX 222 (trade name, manufactured by Merck Ltd.) may be used.

After a mixture containing a phase transfer catalyst, [$^{18}F$]fluoride ions and potassium ions is obtained, a reaction solution containing the mixture and the labeling precursor compound TATM, is prepared.

The process according to the present invention is characterized in that this step for preparing a reaction solution is carried out under a hermetic condition.

This procedure will be explained with reference to Figure 1 as follows. First, the valve (100g) opened during the heat/ evaporation step is now closed, and the vessel (5) is connected with the reaction vessel (1) by operating the three-way stopcocks (100c and 100d). Next, the TATM solution in the vessel (5) is introduced into the reaction vessel (1) by pressurizing the vessel (5) by use of the helium cylinder (6). When the introduction of the TATM solution into the reaction vessel (1) is completed, the pressurization with the helium gas is stopped, and the flow path leading to vessels from the vessel (1) is closed by operating the three-way stopcock (100d).

By the above-mentioned series of operations, the introduction of the TATM solution into the reaction vessel (1) can be performed under a so-called hermetic condition.

After the introduction of the TATM solution into the reaction vessel (1) is completed, the above reaction solution is given a reaction condition by heating with the heating apparatus (7) while the hermetic state is kept, whereby $^{18}F$-TAFDG is synthesized through a nucleophilic substitution reaction. As a reaction condition, a known condition may be used; preferably the temperature of 75-90°C is used. As a reaction time, a known condition may be used; 5-10 minutes may be appropriate in the case where the reaction temperature is 75-90°C ("Manufacture and Quality Control of Radioactive Agents for PET (PET-you Houshasei Yakuzai no Seizou oyobi Hinshitu Kanri in Japanese) - A Guideline to Synthesis and Clinical Use - 2nd Ed." edited by PET Kagaku Workshop). Here, attention must be paid because the longer the reaction time is, the more the above described nucleophilic reaction proceeds, but concurrently radioactive fluorine dacays, thereby causing decrease of total yield.

After the synthesis of $^{18}F$-TAFDG is completed, $^{18}F$-FDG is obtained by acid hydrolysis followed by column purification.

First, by opening the valve (100g) and heating with the heating apparatus (7), acetonitrile is evaporated to exsiccate the $^{18}F$-TAFDG. Then, the vessel (8) charged with hydrochloric acid is connected with the vessel (1) by operating the three-way stopcocks (100d and 100e). And, hydrochloric acid in the vessel (8) is introduced into the vessel (1) using the helium cylinder (6). By adjusting the three-way stopcock (100d) accompanied by closing the valve (100g), the vessel (1) is conditioned to be airtight again and heated with the heating apparatus (7), thereby causing acid hydrolysis. Amount of the acid and heating condition to be used here may be known ones ("Manufacture and Quality Control of Radioactive Agents for PET (PET-you Houshasei Yakuzai no Seizou oyobi Hinshitu Kanri in Japanese) - A Guideline to Synthesis and Clinical Use - 2nd Ed." edited by PET Kagaku Workshop).

After the completion of hydrolysis, the vessel (1) is connected to the purification column (9) by adjusting the three-way stopcocks (100e and 100d) and a valve (100f) is opened. While this state is kept, the vessel (1) is pressurized with the helium cylinder (6), so that the reaction solution in the vessel (1) is passed through the purification column (9) and collected in the vessel (10) to give $^{18}F$-FDG. The purification process may be a known one ("Manufacture and Quality Control of Radioactive Agents for PET (PET-you Houshasei Yakuzai no Seizou oyobi Hinshitu Kanri in Japanese) - A Guideline to Synthesis and Clinical Use - 2nd Ed." edited by PET Kagaku Workshop).

EXAMPLES

Hereinafter, the present invention will be described in further detail with reference to Examples; however, it should be understood that the details of the Examples are not intended to limit the present invention.

Comparative Example 1

For the purpose of comparing yield of production, synthesis of $^{18}F$-FDG was performed by the following method.

$H_2{}^{18}O$ enriched water was subjected to proton bombardment to obtain [$^{18}F$]fluoride ions in a form of a target water containing [$^{18}F$]fluoride ions. This target water containing [$^{18}F$]fluoride ions was measured for the amount of radioactivity using CRC-712X (trade name, manufactured by CAPINTEC, Inc.) (denoted as the amount of radioactivity A). Then, after this target water was passed through an anion exchange column and [$^{18}F$]fluoride ions were collected by adsorption, a potassium carbonate solution was passed through the column to elute [$^{18}F$]fluoride ions into the reaction vessel. To this reaction vessel, a solution of CRYPTOFIX 222 (trade name, manufactured by Merck Ltd.) in acetonitrile was added, heated and evaporated to dryness to prepare a mixture containing [$^{18}F$]fluoride ions, potassium ions and a phase transfer catalyst (CRYPTOFIX 222, trade name, manufactured by Merck Ltd.).

One mL of the solution of TATM in acetonitrile (concentration: 20 mg/mL) was added to the reaction vessel while the reaction vessel was kept open to prepare a reaction solution. Then, the reaction vessel was made airtight by closing the apertures, and a labeling reaction was performed by heating at 85°C for 5 minutes using the heating block. After the reaction was completed, the vessel was made open and heated further at 85°C for 5 minutes to evaporate the solvent. The residue was supplemented with 1 mol/mL hydrochloric acid and heated to perform deprotection, and was purified with a purification column to yield $^{18}$F-FDG. The obtained $^{18}$F-FDG was measured for the amount of radioactivity using CRC-712X (trade name, manufactured by CAPINTEC, Inc.) (denoted as the amount of radioactivity B). The procedure of the synthesis was repeated 25 times.

The obtained reaction product was subjected to TLC analysis under the condition described hereunder to obtain an area percentage of $^{18}$F-FDG as radiochemical purity, and thereby it was confirmed that $^{18}$F-FDG was obtained in a radiochemical purity of 95 % or more.

TLC analysis condition:

TLC plate: Silica Gel 60 $F_{254}$ (trade name, manufactured by Merck Ltd.)
Mobile phase: chloroform/ethyl acetate = 80/20
Detector: JTC-601 (trade name, manufactured by Aloka Co.

Ltd.)

Yield of $^{18}$F-FDG synthesis was determined according to the following equation (1) using the measured amounts of radioactivity A and radioactivity B. Here, the amounts of radioactivity A and radioactivity B corrected for decay in consideration of measurement time were used in the calculation.

$$Yield\ of\ ^{18}F - FDG\ synthesis\ (\%) = \frac{B}{A} \times 100 \quad (1)$$

The results were shown in Figure 2. The yield of $^{18}$F-FDG synthesis was 58.41 ± 10.15 %. In the method in which the TATM solution was added in an open system, variation of the yield of $^{18}$F-FDG synthesis was large and the average value was not considered to be sufficiently high.

Example 1

The mixture containing [$^{18}$F]fluoride ions, potassium ions and a phase transfer catalyst (CRYPTOFIX 222, trade name, manufactured by Merck Ltd.) was prepared in the reaction vessel in the same way as Comparative Example 1. This reaction vessel was closed except the flow inlet for the solution of TATM, and 1 mL of the solution of TATM in acetonitrile (concentration: 20 mg/mL) was introduced into the vessel through the flow inlet by pressurizing with the helium cylinder.

Then, $^{18}$F-FDG was synthesized by performing the reaction and purification in the same way as Comparative Example 1. The obtained $^{18}$F-FDG was measured for yield of $^{18}$F-FDG synthesis using a method similar to that in Comparative Example 1. Also, radiochemical purity was obtained in the same way as Comparative Example 1, and thereby it was confirmed that $^{18}$F-FDG was obtained in a radiochemical purity of 95 % or more. The procedure of synthesis was repeated 9 times.

The results are shown in Figure 2. The yield of $^{18}$F-FDG synthesis according to the process of the present invention was 75.27 ± 3.10 %, and it was shown that $^{18}$F-FDG was obtained in a higher yield and the variation of the yield was smaller compared with the conventional method (Comparative Example 1).

As described above, it was shown that the yield of $^{18}$F-FDG synthesis can be improved and stabilized by using the production process according to the present invention.

INDUSTRIAL APPLICABILITY

The process for production of a compound labeled with radioactive fluorine according to the present invention can be employed in producing a radiopharmaceutical $^{18}$F-FDG, and is useful in the field of nuclear medicine examination.

BRIEF EXPLANATION OF THE DRAWINGS

Figure 1 depicts a schematic diagram illustrating an example of a synthesis apparatus for performing the process according to the present invention; and

Figure 2 shows the yield of $^{18}$F-FDG synthesis according to the process of the present invention and the yield of $^{18}$F-FDG synthesis according to a conventional method.

EXPLANATION OF SYMBOLS

1 reaction vessel,
2 anion-exchange column,
3-5, 8 reagent vessel,
6 helium cylinder,
7 heating apparatus,
9 purification column,
10 $^{18}$F-FDG collecting vessel,
11 $^{18}$O enriched water recovering vessel,
12 $^{18}$F storing vessel,
100a-e, h three-way stopcock, and
100f, g valve for opening and closing.

**Claims**

1. A process for production of a compound labeled with radioactive fluorine, which comprises the steps of:

preparing a reaction solution by adding, under a hermetic condition, 1,3,4,6-tetra-O-acetyl-2-O-trifluoromethanesulfonyl-β-D-mannopyranose and an inert organic solvent to a mixture containing [$^{18}$F]fluoride ions, a phase transfer catalyst and potassium ions;

giving a reaction condition to said reaction solution under a hermetic condition to obtain 1,3,4,6-tetra-O-acetyl-2-[$^{18}$F]fluoro-2-deoxyglucose; and

subjecting the obtained 1,3,4,6-tetra-O-acetyl-2-[$^{18}$F]fluoro-2-deoxyglucose to a deprotection process to obtain 2-[$^{18}$F]fluoro-2-deoxy-D-glucose.

2. A process for production of a compound labeled with radioactive fluorine, which comprises the steps of:

obtaining a mixture containing [$^{18}$F]fluoride ions, a phase transfer catalyst and potassium ions in a vessel connected to one or more flow paths;

adding 1,3,4,6-tetra-O-acetyl-2-O-trifluoromethanesulfonyl-β-D-mannopyranose and an inert organic solvent to said mixture through a path for introducing a raw material in a state in which the flow paths other than the flow path for introducing a raw material are closed, to prepare a reaction solution;

heating said reaction solution under a hermetic condition in a state in which all the flow paths of said vessel are closed, to obtain 1,3,4,6-tetra-O-acetyl-2-[$^{18}$F]fluoro-2-deoxyglucose; and

subjecting the obtained 1,3,4,6-tetra-O-acetyl-2-[$^{18}$F]fluoro-2-deoxyglucose to a deprotection process to obtain 2-[$^{18}$F]fluoro-2-deoxy-D-glucose.

3. A process for production of a compound labeled with radioactive fluorine, which comprises the steps of:

obtaining a mixture containing [$^{18}$F]fluoride ions, a phase transfer catalyst and potassium ions in a vessel having one aperture;

adding 1,3,4,6-tetra-O-acetyl-2-O-trifluoromethanesulfonyl-β-D-mannopyranose and an inert organic solvent to said mixture through said aperture to prepare a reaction solution;

heating said reaction solution under a hermetic condition whilst said aperture is closed, to obtain 1,3,4,6-tetra-O-acetyl-2-[$^{18}$F]fluoro-2-deoxyglucose; and

subjecting the obtained 1,3,4,6-tetra-O-acetyl-2-[$^{18}$F]fluoro-2-deoxyglucose to a deprotection process to obtain 2-[$^{18}$F]fluoro-2-deoxy-D-glucose.

4. The process for production of a compound labeled with radioactive fluorine, according to any one of claims 1 to 3, wherein said step of preparing a reaction solution is performed by adding a solution of 1,3,4,6-tetra-O-acetyl-2-O-trifluoromethanesulfonyl-β-D-mannopyranose in an inert organic solvent to said mixture by pressurizing the solution with an inert gas.

FIG. 1

FIG. 2

## INTERNATIONAL SEARCH REPORT

| International application No. |
| --- |
| PCT/JP2006/323932 |

A.  CLASSIFICATION OF SUBJECT MATTER
*C07H5/02*(2006.01)i, *A61K51/00*(2006.01)n, *C07B61/00*(2006.01)n

According to International Patent Classification (IPC) or to both national classification and IPC

B.  FIELDS SEARCHED

Minimum documentation searched (classification system followed by classification symbols)
C07H5/02, C07H13/06, A61K51/00, C07B61/00

Documentation searched other than minimum documentation to the extent that such documents are included in the fields searched
Jitsuyo Shinan Koho          1922-1996    Jitsuyo Shinan Toroku Koho    1996-2007
Kokai Jitsuyo Shinan Koho    1971-2007    Toroku Jitsuyo Shinan Koho    1994-2007

Electronic data base consulted during the international search (name of data base and, where practicable, search terms used)
CAplus(STN), CASREACT(STN), REGISTRY(STN)

C.  DOCUMENTS CONSIDERED TO BE RELEVANT

| Category* | Citation of document, with indication, where appropriate, of the relevant passages | Relevant to claim No. |
| --- | --- | --- |
| Y<br>A | JP 64-75498 A  (The Japan Steel Works, Ltd.),<br>22 March, 1989 (22.03.89),<br>(Family: none) | 1-3<br>4 |
| Y<br>A | JP 5-345731 A  (NKK Corp.),<br>27 December, 1993 (27.12.93),<br>(Family: none) | 1-3<br>4 |
| Y<br>A | JP 8-201588 A  (Nobuhiko NAKAZAWA),<br>09 August, 1996 (09.08.96),<br>& US 5759513 A | 1-3<br>4 |

☐  Further documents are listed in the continuation of Box C.          ☐  See patent family annex.

| | |
| --- | --- |
| *    Special categories of cited documents:<br>"A"  document defining the general state of the art which is not considered   to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search<br>05 February, 2007 (05.02.07) | Date of mailing of the international search report<br>13 February, 2007 (13.02.07) |
| --- | --- |
| Name and mailing address of the ISA/<br>Japanese Patent Office | Authorized officer |
| Facsimile No. | Telephone No. |

Form PCT/ISA/210 (second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 6157572 A

### Non-patent literature cited in the description

- **HAMACHER K. ; COENEN H.H. ; STOCKLIN G.** Efficient Stereospecific Synthesis of No-carrier-added-2-[18F]fluoro-2-deoxy-D-glucose Using Aminopolyether Supported Nucleophilic Substitution. *J. Nucl. Med.*, 1986, vol. 27 (2), 235-238
- Computer-aided Synthesis (CAS) of No-carrier-added-2-[18F]Fluoro-2-deoxy-D-glucose: an Efficient Automated System for the Aminopolyether-supported Nucleophilic Fluorination. **K. HAMACHER et al.** Applied Radiation and Isotopes, (Great Britain. Pergamon Press, 1990, vol. 41, 49-55
- Ion Chromatographic Analysis of High Specific Activity 18FDG Preparations and Detection of the Chemical Impurity 2-Deoxy-2-chloro-D-glucose. **DAVID L. ALEXOFF et al.** Applied Radiation and Isotopes, (Great Britain. Pergamon Press, 1992, vol. 43, 1313-1322
- Manufacture and Quality Control of Radioactive Agents for PET (PET-you Houshasei Yakuzai no Seizou oyobi Hinshitu Kanri in Japanese. A Guideline to Synthesis and Clinical Use
- Manufacture and Quality Control of Radioactive Agents for PET. A Guideline to Synthesis and Clinical Use